# EUROPEAN PATENT SPECIFICATION

(11) **EP 4 275 703 B1**
(45) Date of publication and mention of the grant of the patent: **06.08.2025**
(21) Application number: 22908810.9
(22) Date of filing: 09.12.2022
(51) Int. Cl.: C07K 16/28, A61P 9/10, A61P 9/00, A61K 39/00, C12N 5/077

(54) **PD1 INHIBITOR FOR USE FOR TREATING MYOCARDIAL INFARCTION BY INHIBITION OF CARDIAC FIBROBLAST TRANSDIFFERENTIATION**
PD1-INHIBITOR ZUR VERWENDUNG ZUR BEHANDLUNG VON MYOKARDINFARKT DURCH HEMMUNG DER TRANSDIFFERENZIERUNG VON KARDIALEN FIBROBLASTEN
INHIBITEUR DE PD1 DESTINÉ À ÊTRE UTILISÉ POUR TRAITER UN INFARCTUS DU MYOCARDE PAR INHIBITION DE LA TRANSDIFFÉRENCIATION DES FIBROBLASTES CARDIAQUES

(30) Priority: 29.03.2022 CN 202210323171
(43) Date of publication of application: 15.11.2023
(73) Proprietor: Zhejiang University, Hangzhou, Zhejiang 310058 (CN)
(72) Inventor: HU, Xinyang, Hangzhou, Zhejiang 310058 (CN); WANG, Jian'an, Hangzhou, Zhejiang 310058 (CN); CHEN, Xiaoying, Hangzhou, Zhejiang 310058 (CN); KE, Changle, Hangzhou, Zhejiang 310058 (CN)
(74) Representative: Elzaburu S.L.P.
(86) International application number: PCT/CN2022/137907
(87) International publication number: WO 2023/185085

(56) References cited:
- WO-A1-2020/236078
- CN-A- 106 687 125
- CN-A- 114 984 219
- US-A1- 2020 164 031
- US-A1- 2020 262 923
- US-A1- 2021 353 679
- HEINZERLING LUCIE ET AL: "Cardiotoxicity associated with CTLA4 and PD1 blocking immunotherapy", vol. 4, no. 1, 1 December 2016 (2016-12-01), GB, XP093145676, ISSN: 2051-1426, Retrieved from the Internet <URL:https://www.ncbi.nlm.nih.gov/pmc/articles/PMC4986340/pdf/40425_2016_Article_152.pdf> DOI: 10.1186/s40425-016-0152-y

## Description

### Technical Field

The present invention relates to the field of biomedicine, and in particular relates to a PD 1 inhibitor for use for treating myocardial infarction by inhibition of cardiac fibroblast transdifferentiation.

The present invention also relates to an *in vitro* method for inhibiting cardiac fibroblast transdifferentiation using the same PD1 inhibitor.

### Background

Myocardial infarction is a common cardiovascular disease that poses a serious threat to human health. Although many patients with acute myocardial infarction have been able to survive with the improvement of medical technology in recent years, myocardial remodeling after myocardial infarction may lead to heart failure, and severe heart failure is the main cause of death for patients. Each phase after myocardial infarction has its own characteristic pathophysiological changes, and the infarction process involves mutual interaction and joint participation of a variety of cells, including a series of pathophysiological processes such as early massive myocardial cell death, immune cell infiltration, angiogenesis, fibroblast activation, myocardial compensatory hypertrophy, and chronic pathological myocardial remodeling. In recent years, a series of studies have shown that immune cells participate in the whole process of chronic myocardial remodeling caused by myocardial infarction, and it is an important target to improve the prognosis of myocardial infarction by controlling angiogenesis and fibroblast activation with immune cells.

PD1/PD-L1 is a second stimulatory signaling pathway other than a T cell antigen receptor (TCR). Blocking the signaling activity of PD1 can reactivate the biological activity of depleted T cells and exert the immune activity thereof, which is also a research hotspot in tumor therapy at present. However, the regulatory mechanism of the PD1/PD-L1 signaling pathway on immune cell functions in non-tumor lesions is unclear.

In addition, the biological effects of PD1 inhibitors among different types of diseases and in different tissues and organs are obviously different. For example, in the nervous system, PD1 inhibitors can weaken the analgesic effect of morphine; in a process of treating tumors, PD1 inhibitors can cause side effects in the nervous system, mainly neuromuscular lesions; and in terms of Alzheimer's disease, studies have shown that blocking the PD1 signaling pathway can improve the symptom. This indicates that the ultimate effects of PD1 inhibitors vary significantly among different diseases and in different organs and have no significant similarity. In existing reports, there are still few reports on application of PD1 inhibitors in cardiac diseases. In myocardial infarction, excessive fibrosis (excessive transdifferentiation) of cardiac fibroblasts can worsen cardiac function. Therefore, it is a potentially effective treatment approach to find drugs capable of inhibiting transdifferentiation of cardiac fibroblasts for treating diseases related to excessive fibrosis of cardiac fibroblasts.

### Summary

To solve the above technical problems, the present invention provides a PD1 inhibitor for use for treating myocardial infarction by inhibition of cardiac fibroblast transdifferentiation, wherein the PD1 inhibitor is nivolumab. The invention is defined by the appended claims.

The present disclosure found through cell and animal experiments that the PD1 inhibitor can reduce the transdifferentiation level of cardiac fibroblasts after myocardial infarction. The present invention provides an *in vitro* method for inhibiting cardiac fibroblast transdifferentiation using a PD1 inhibitor, wherein the PD1 inhibitor is nivolumab.. Preferably, the inhibitor for cardiac fibroblast transdifferentiation is used as a tool drug for scientific studies or a drug for treating myocardial infarction.

Preferably, the PD1 inhibitor is selected from nivolumab, Pembrolizumab and T-drug (Tecentrip).

Further preferably, the PD1 inhibitor is nivolumab.

Preferably, the tool drug or drug contains the PD1 inhibitor, and a pharmaceutically acceptable carrier and/or excipient.

Preferably, the tool drug or drug is an injection preparation.

Further preferably, the injection preparation is an intravenous injection preparation.

Compared with the prior art, the present disclosure has the following technical effects: After mouse models of heart failure after myocardial infarction are established and intervened using a PD1 inhibitor, the heart function is evaluated by echocardiography and Sirius red, and it is found that the PD1 inhibitor can reduce the transdifferentiation level of cardiac fibroblasts after myocardial infarction.

Any references in the description to methods of treatment refer to the PD1 inhibitor of the present invention for use for treating myocardial infarction by inhibition of cardiac fibroblast transdifferentiation, wherein the PD1 inhibitor is nivolumab.

### Brief Description of Drawings

FIG. 1 is a diagram showing the effect of coculture of PD1+T cells and fibroblasts on the transdifferentiation level of fibroblasts in an example of the present disclosure.
FIG. 2 is a diagram showing the effect of PD1+T cells on cardiac inflammatory response and fibrosis in an example of the present disclosure.
FIG. 3 is a diagram showing the effect of a PD1 inhibitor on cardiac function after myocardial infarction in an example of the present disclosure.
FIG. 4 is a diagram showing the effect of a PD1 inhibitor on fibrosis after myocardial infarction in an example of the present disclosure.
FIG. 5 is a diagram showing the effect of a PD1 inhibitor on inhibiting fibrosis by inhibiting secretion of inflammatory factors in an example of the present disclosure.
FIG. 6 is a diagram showing the effect of a PD1 inhibitor on the fibrosis level of a remote area in non-human primate cynomolgus monkeys after myocardial infarction in an example of the present disclosure.

### Detailed Description

The present invention will be further described in conjunction with examples below.

### General examples

The present invention provides a PD1 inhibitor for use for treating myocardial infarction by inhibition of cardiac fibroblast transdifferentiation. The PD1 inhibitor can be specifically used as a tool drug for scientific studies or a drug for treating myocardial infarction. Preferably, the PD1 inhibitor is selected from nivolumab, Pembrolizumab and T-drug (Tecentrip); and further preferably, the PD1 inhibitor is nivolumab.

Preferably, the tool drug or drug contains the PD1 inhibitor, and a pharmaceutically acceptable carrier and/or excipient. Further preferably, the tool drug or drug is an injection preparation, and most preferably, an intravenous injection preparation.

### Example 1 (in vitro cell experiment)

(1) Neonatal rat cardiac fibroblasts were isolated. The first generation neonatal rat cardiac fibroblasts were conventionally cultured in a low sugar dulbecco's modified eagle medium (DMEM) containing 10% FBS (v/v) in a mixed gas of 5% CO₂ and 95% air by volume at 37°C. Once confluent, 0.25% trypsin (w/v)-0.02% ethylenediaminetetraacetic acid (EDTA) (w/v) was used for digestion and passage.
(2) After the neonatal rat cardiac fibroblasts grew to a confluence of 50%, the conventional cell culture medium containing 10% FBS was removed. The neonatal rat cardiac fibroblasts were washed using PBS 3 times or more, and cultured in a cell culture incubator (Forma 3111, Thermo Fisher, USA) containing 5% CO₂ and having a saturated humidity at 37°C for 1 day, and the culture medium was changed daily.
(3) PD1+T cells and PD1-T cells in myocardial infarction were sorted by flow cytometry. T cells were collected 7 days after myocardial infarction. Red blood cells were lysed with a red blood cell lysate (Solarbio) for 10 minutes. The cell lysates were centrifuged at room temperature, 1200 rpm for 5 minutes. The supernatant was discarded. A PBS buffer solution was added for centrifugation and washing 2 times. The cells were resuspended in a PBS buffer solution containing 1% BSA to prepare 5×10⁵/100 µl cell suspensions. The cell suspensions were blocked at room temperature for 20 minutes. Then anti-mouse CD45, CD3, and CD279 antibodies and the isotype control antibodies thereof (BIOLEGEND, USA) were sequentially added. The cells were incubated at room temperature in dark for 20 minutes, and centrifuged at 1500 rpm for 5 minutes to remove the antibodies. A PBS buffer solution was added for centrifugation and washing 2 times. Finally, 500 µl of PBS buffer solution was added for resuspension and sorting by flow cytometry (BD, USA).
(4) The sorted T cells were cocultured with the neonatal rat cardiac fibroblasts. The T cells were placed in a Tran'swell insert (Costar, USA, pore size 0.4 µm) at a concentration of 2*10⁵/ml in a 1640 culture medium. A PD1 inhibitor (BloX cell) (10 ug/ml) was added to the supernatant of the tran'swell insert for treatment for 48 hours at the same time. The neonatal rat cardiac fibroblasts were placed in a lower chamber of the tran'swell plate.
(5) After 48 hours of coculturing, the neonatal rat cardiac fibroblasts were collected from the lwer chamber, and the transdifferentiation level of the neonatal rat cardiac fibroblasts was measured by Western Blot.

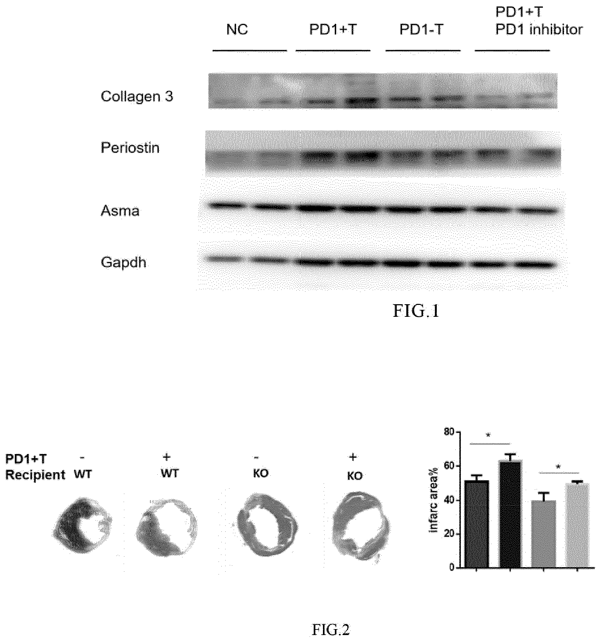

As shown in FIG. 1, PD1+T cells can obviously promote high expression of transdifferentiation proteins Collagen 3, Periostin, and Asma in neonatal rat cardiac fibroblasts compared to PD1-T cells. The PD1 inhibitor can inhibit transdifferentiation of the neonatal rat cardiac fibroblasts induced by the PD1+T cells. Therefore, the PD1+T cells are more capable of promoting the transdifferentiation of the neonatal rat cardiac fibroblasts. Example 2 (in vivo experiment in mice)
(1) Medication of a model for treating myocardial infarction in mice includes: intraperitoneal injection of a PD1 inhibitor (BloX cell) (400 ug/20 g) was conducted 24 hours in advance before a myocardial infarction model was established, and depilation and skin preparation were conducted on the mouse's chest. Establishment of a myocardial infarction model: a mouse was anesthetized using 4% pentobarbital (10 mg/0.8 ml) and fixed on a mouse board under anaesthetic, followed by tracheal intubation and connection to a ventilator. The ventilator was adjusted to a frequency of 98 beats per minute and a tidal volume of 1.2-1.5 ml. The surface skin of the left chest of the mouse was cut open using scissors, the chest muscles were bluntly dissected using tweezers and cut along the left 3-4 ribs using scissors, and then the ribs were gradually opened using a small animal chest expander to fully expose the heart. The pericardium was dissected, and it could be seen that the left anterior descending coronary artery traverses between the left atrial appendage and the pulmonary conus. The anterior descending artery was ligated using a 7-0 prolene thread 2 mm below the left atrial appendage. After the myocardial infarction models were established, intraperitoneal injection of the PD1 inhibitor (BIoX cells) was conducted every 3 days, with a total course of 28 days. The cardiac function was measured by echocardiography at day 3, day 7, day 14, and day 28 after myocardial infarction.
(2) 28 days after the myocardial infarction, a mouse was anesthetized using 4% pentobarbital (10 mg/0.8 ml) and fixed on a mouse board. The abdomen was opened to expose the inferior vena cava, and 100 ul of potassium chloride (5 mg/ml) was injected intravenously to arrest the heart in diastole. The chest was opened, and the heart at the site of the infarction was gently peeled off from the ribs using a cotton swab. The right atrial appendage of the heart was cut open using scissors. A PBS buffer solution was injected from the left ventricular apex into the mouse heart using a 1 ml syringe for perfusion until the color of the mouse liver and lungs turned white. The heart was isolated along the root of the aorta and embedded in 30% sucrose for tissue freezing and pathological sectioning. Pathological sections were stained using Masson trichrome and photographed using a stereomicroscope to evaluate the myocardial infarct area. Blue and red intima and adventitia lengths were drawn using Imagepro plus software. The myocardial infarct area is calculated according to: (myocardial infarction intima+adventitia)/(total intima+adventitia). 4 sections were taken from each heart for statistical analysis.

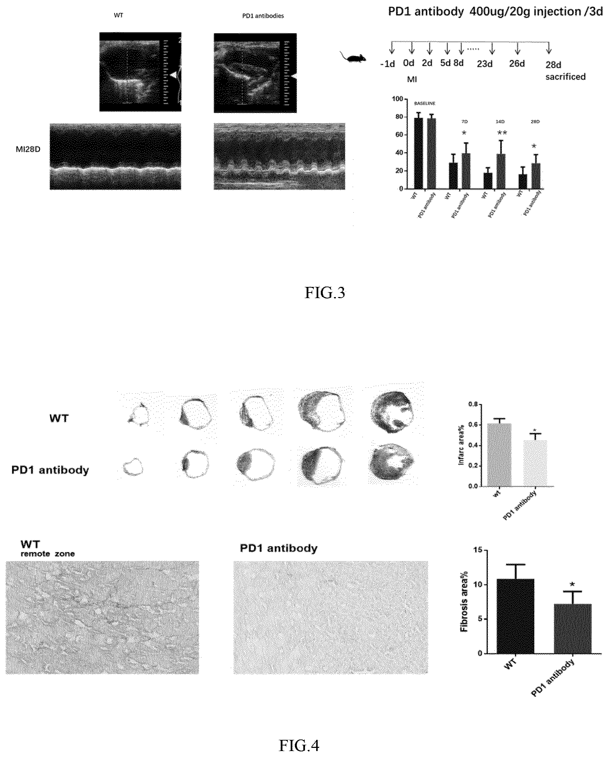

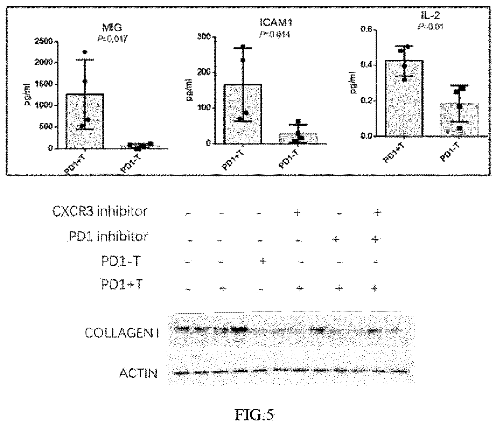

As shown in FIG. 2, reinfusion of PD1+T obviously increases the infarct area of the heart and worsens the cardiac function. FIG. 3 shows that after the myocardial infarction models are established, treatment with the PD1 inhibitor improves the cardiac systolic function. FIG. 4 shows that after the myocardial infarction models are established, treatment with the PD1 inhibitor reduces the myocardial infarct area and also reduces fibrosis in the remote area of the heart after myocardial infarction. This indicates that the PD1 inhibitor can inhibit myocardial infarction inflammatory response and myocardial fibrosis after myocardial infarction. The mechanism exploration is shown in FIG. 5. By sorting PD1+T and PD1-T cells for inflammatory factor sequencing, it is found that the PD1+T cells overexpress the molecules MIG that promote fibroblast transdifferentiation. Furthermore, by inhibiting CXCR3 using MIG to block the function of MIG, it is found that blocking of inflammatory factors secreted by the PD1+T cells can obviously improve the transdifferentiation level of fibroblasts. This indicates that the PD1 inhibitor can further inhibit the transdifferentiation of cardiac fibroblasts by inhibiting secretion of the inflammatory factors.

### Example 3 (in vivo experiment in cynomolgus monkeys)

(1) Establishment of a cynomolgus monkey myocardial infarction model: after skin preparation and induced anesthesia, a cynomolgus monkey was fixed in a supine position on an operating table, followed by tracheal intubation and connection to a ventilator for assisted ventilation. The skin of the surgical field was disinfected using iodophor. The chest was opened layer by layer. The pericardium was dissected from the third intercostal space to expose the heart. The anterior descending coronary artery was permanently ligated using a 6-0 nylon thread. The color of the area below the ligation immediately turned pale, and local myocardial activity decreased. At this point, the myocardial infarction model had been successfully established. After the operation, the pericardium and pleura were sutured using a 4-0 polyethylene suture, the sternum was sutured using a 10-0 silk suture, and then the skin incision was sutured using a 1-0 silk suture. After spontaneous breathing was recovered, the endotracheal cannula was removed from the monkey.
(2) Groups of the in vivo animal experiment: a. MI (myocardial infarction) group, with intravenous injection of physiological saline before MI modeling; b. MI+(nivolumab, 10 mg/kg), administered 24 hours before modeling and at day 14 after modeling by intravenous injection.
(3) Index evaluation:
   A. Cardiac function evaluation: On the 1 day before modeling, day 3 and day 28 after nivolumab treatment, the cardiac function was evaluated by measuring the left ventricular ejection fraction (EF) and left ventricular fractional shortening (FS) by cardiac MRI and echocardiography for each group of monkeys, the dilatation of heart was evaluated by the left ventricular end systolic diameter and end diastolic diameter, and the myocardial infarct area was evaluated by delayed cardiac MRI imaging scan.
   B. Before the operation, and at day 1, day 3, day 7, day 14, and day 28 after injection of the inhibitor, blood samples were collected to measure blood routine, myocardial zymogram, liver and kidney function, thyroid function, and blood concentration of the inhibitor, to further evaluate the safety of the inhibitor in treating myocardial infarction.
   C. The cardiac tissue was taken at day 28. The heart was equalized into 5 parts from the apex to the fundus, and photographed by a light microscope to evaluate the myocardial infarct area. Then, the heart was sectioned into equal parts according to the infarct area, peri-infarct area, and infarction remote area, and stored in liquid nitrogen, formalin, and 30% sucrose, respectively.

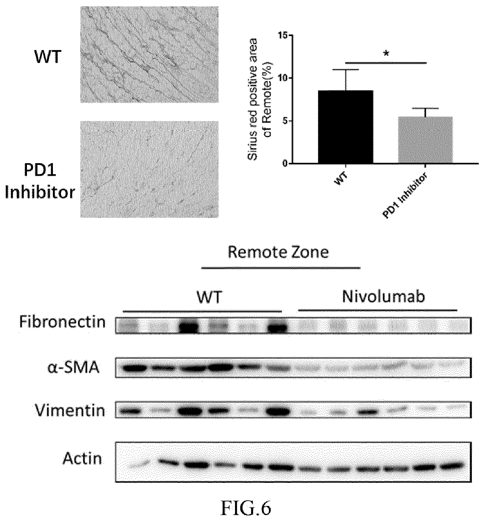

The status of cardiac tissue fibrosis in the above groups was compared by Western blot (asma, periostin and fibronectin), Masson, Sirius red, and other methods, to evaluate the effect of the inhibitor on cardiac fibrosis during the treatment of myocardial infarction. FIG. 6 shows that in the cynomolgus monkey models, when the myocardial infarction models are established and administered with the PD1 inhibitor at the same time, it is found that the PD1 inhibitor can obviously improve the cardiac function of the cynomolgus monkey after myocardial infarction. By evaluating the fibrosis using Sirius red in the remote area of myocardial infarction, it is found that the PD1 inhibitor can improve the level of fibrosis in the remote area after myocardial infarction. Western blot showed an obvious decrease in the transdifferentiation expression levels of asma, periostin and fibronectin in the remote area of myocardial infarction after administration of the PD1 inhibitor, indicating that the PD1 inhibitor could also inhibit excessive fibrosis after cardiac remodeling in the cynomolgus monkey.

The raw materials and equipment used in the present disclosure, unless otherwise specified, are those commonly used in the art. The methods used in the present disclosure, unless otherwise specified, are all conventional methods in the art.

## Claims

1. A PD1 inhibitor for use for treating myocardial infarction by inhibition of cardiac fibroblast transdifferentiation, wherein the PD1 inhibitor is nivolumab.

2. A drug comprising a PD1 inhibitor for use according to claim 1 and a pharmaceutically acceptable excipient.

3. The drug for use of claim 2, which is an injection preparation.

4. The drug for use of claim 3, wherein the injection preparation is an intravenous injection preparation.

5. An *in vitro* method for inhibiting cardiac fibroblast transdifferentiation using a PD1 inhibitor, wherein the PD1 inhibitor is nivolumab.

## Patentansprüche

1. PD1-Inhibitor zur Verwendung zur Behandlung von Myokardinfarkt durch Hemmung der Transdifferenzierung von kardialen Fibroblasten, wobei der PD1-Inhibitor Nivolumab ist.

2. Medikament, umfassend einen PD1-Inhibitor zur Verwendung nach Anspruch 1 und einen pharmazeutisch zulässigen Hilfsstoff umfasst.

3. Medikament zur Verwendung nach Anspruch 2, das ein Injektionspräparat ist.

4. Medikament zur Verwendung nach Anspruch 3, wobei das Injektionspräparat ein intravenöses Injektionspräparat ist.

5. *In-vitro-Verfahren* zur Hemmung der Transdifferenzierung von kardialen Fibroblasten unter Verwendung eines PD1-Inhibitors, wobei der PD1-Inhibitor Nivolumab ist.

## Revendications

1. Un inhibiteur de PD1 à utiliser pour traiter l'infarctus du myocarde par inhibition de la transdifférenciation des fibroblastes cardiaques, dans lequel l'inhibiteur de PD1 est le nivolumab.

2. Un médicament comprenant un inhibiteur de PD1 à utiliser selon la revendication 1 et un excipient pharmaceutiquement acceptable.

3. Le médicament à utiliser selon la revendication 2, qui est une préparation pour injection.

4. Le médicament à utiliser selon la revendication 3, pour lequel la préparation pour injection est une préparation pour injection intraveineuse.

5. Méthode *in vitro* pour inhiber la transdifférenciation des fibroblastes cardiaques à l'aide d'un inhibiteur de PD1, dans laquelle l'inhibiteur de PD1 est le nivolumab.
